# EUROPEAN PATENT SPECIFICATION

(11) **EP 3 170 557 B1**
(45) Date of publication and mention of the grant of the patent: **06.05.2020**
(21) Application number: 15195711.5
(22) Date of filing: 20.11.2015
(51) Int. Cl.: C07C 209/26, C07C 211/04, B01J 31/24

(54) **METHOD FOR THE SYNTHESIS OF TRIMETHYLAMINE**
VERFAHREN ZUR SYNTHESE VON TRIMETHYLAMIN
PROCÉDÉ POUR LA SYNTHÈSE DE LA TRIMÉTHYLAMINE

(43) Date of publication of application: 24.05.2017
(73) Proprietor: Rheinisch-Westfälische Technische Hochschule (RWTH) Aachen, 52056 Aachen (DE)
(72) Inventor: Klankermayer, Jürgen, 45259 Essen (DE); Leitner, Walter, 52074 Aachen (DE); Kassem, Beydoun, 52074 Aachen (DE)
(74) Representative: Michalski Hüttermann & Partner Patentanwälte mbB

(56) References cited:
- EP-A1- 2 821 397
- KASSEM BEYDOUN ET AL: "Selective Synthesis of Trimethylamine by Catalytic N -Methylation of Ammonia and Ammonium Chloride by utilizing Carbon Dioxide and Molecular Hydrogen", CHEMCATCHEM, vol. 8, no. 1, 2 December 2015 (2015-12-02), pages 135-138, XP055264637, DE ISSN: 1867-3880, DOI: 10.1002/cctc.201501116
- OLIVIER JACQUET ET AL: "CO2 as a C1-building block for the catalytic methylation of amines", CHEMICAL SCIENCE, ROYAL SOCIETY OF CHEMISTRY, UNITED KINGDOM, vol. 4, 1 January 2013 (2013-01-01), pages 2127-2131, XP002716712, ISSN: 2041-6520, DOI: 10.1039/C3SC22240C [retrieved on 2013-02-25]
- GREDIG S V ET AL: "PALLADIUM CATALYZED SYNTHESIS OF METHYLAMINES FROM CARBON DIOXIDE, HYDROGEN AND AMMONIA", CATALYSIS LETTERS, SPRINGER NEW YORK LLC, UNITED STATES, vol. 46, no. 1/02, 1 June 1997 (1997-06-01), pages 49-55, XP000692742, ISSN: 1011-372X, DOI: 10.1023/A:1019085511301

## Description

The present invention relates to a method for synthesizing trimethylamine.

Methylamines, the simplest members of the aliphatic amine family, represent highly important and versatile intermediates within the chemical supply chain. The industrial significance of methylamines is reflected by a worldwide annual production larger than 1.3 × 10⁶ t with a growing rate of 3.5% per annum.

Methylamines are prepared on industrial scale inter alia by the exothermic reaction of ammonia (NH₃) and methanol (CH₃OH) with an amorphous silica-alumina catalyst in fixed-bed reactors at elevated temperatures. However the reaction usually leads to mixed components, the composite equilibria within the reaction network result in complex product mixtures and the three possible methylamines (mono-, di-, and tri methyl amine: MMA, DMA and TMA) are generally produced in moderate selectivity for the individual product, To further complicate things, MMA, DMA, TMA and NH₃ form an azeotropic mixture and the use of zeolites-based catalysts in combination with methanol may result in dimethyl ether (DME) as an additional by-product. Consequently, the downstream purification process is laborious and requires sophisticated separation methods.

Reduction methods are inter alia known from the EP 2 821 397, Jacquet et al, Chemical Science, 2013, 2127-2131 and Gredig et al, Catalysis letters, 1997, 49-55.

Therefore there is a constant need in the art for alternative synthesis methods of trimethylamine and it is an object of the present invention to provide them.

This object is achieved in the present invention by a method for the synthesis of trimethylamine, comprising the step of
a) hydrogenating CO₂ in the presence of ammonia and/or an ammonia salt and in the presence of a Ruthenium-Phosphine-complex, whereby when ammonia is used additionally a Lewis acid is present

The term "hydrogenation" in the sense of the present invention especially means and/or includes the reaction of CO₂ with molecular hydrogen and/or a source of molecular hydrogen.

The term "ammonia and/or an ammonia salt" especially means and/or includes gaseous ammonia or ammonia in solution (in case a solvent is used in step a) or liquid ammonia. Preferred ammonium salts are ammonium halogenides, especially chlorides, bromides, triflates, borates, acetates, phosphates, sulfates "Ammonia and/or an ammonia salt" means especially that a mixture of ammonia and a salt can be present.

The term "phosphine" in the sense of the present invention especially means and/or includes trivalent phosphororganic compounds, especially compounds with the general formula PR¹R²R³, R¹ to R³ being independent from each other an organic residue such as e.g. a substituted or unsubstituted alkyl, aryl and/or heteroaryl.

The term "Ruthenium-Phosphine-complex" especially means and/or includes a ruthenium complex where in the coordination sphere of the ruthenium a trivalent phosphororganic component is present so that a bond (may it be a covalent and/or a coordination bond) between the ruthenium and the trivalent phosphororganic component is formed at least temporarily during the reaction.

Surprisingly it has been found that by doing so it is possible to synthesize trimethylamine in excellent yields. For most applications within the present invention, at least one of the following advantages could be observed:
- The reaction can be performed without the need of sophisticated equipment
- The reaction can be used on an industrial scale as well as on a small scale
- The reaction is be easily adaptable to various applications and their demands
- The high selectivity of the reaction simplifies subsequent down-stream processing

It should be noted that the Ruthenium-Phosphine-complex may be used as a homogenous catalyst or in immobilized form. Also two-phase systems and phase-transfer-catalysis may be used depending on the actual application of the invention. Besides a reaction in batch mode, also a continuous reaction system is possible.

It should furthermore be noted that the Ruthenium-Phosphine-complex may include other ligands such as (but not limited to) carbene, nitrogen containing-ligands such as amines or amides, phosphites, phosphoamidites, phosphoric ethers or esters etc.

According to a preferred embodiment of the present invention, the initial ratio of CO₂ to ammonia/ammonia salt (mol:mol) is ≥ 4:1, preferably ≥ 5:1. It has been shown for many applications that already this modest surplus of CO₂ is sufficient for excellent yields.

However this is not limiting (CO₂ can also be a solvent, as will be described later on), the surplus of CO₂ may be much larger.

According to a preferred embodiment of the invention, the (initial) molar ratio of ruthenium to ammonia/ammonia salt is ≥0.01 to ≤0.1, preferably ≥0.02 to ≤0.05. This has been shown to be advantageous for many applications.

According to a preferred embodiment of the present invention, step a) is performed under acidic conditions. This has been shown to greatly increase the efficiency for most applications within the present invention.

The term "acidic conditions" in the sense of the present invention especially means and/or includes that during the reaction at least temporarily more acid than base is present.

According to the present invention, when ammonia is used additionally a Lewis acid is present. Preferred Lewis acids are selected out of aluminum salts and boranes. Especially preferred are aluminum salts, more preferably aluminum salts of organic acids, most preferred Al(OAc)₃ and /or Al(OTf)₃ or mixtures thereof.

Preferably the concentration of lewis acid to ruthenium (in mol:mol) is ≥ 5:1 to ≤ 20:1, more preferably ≥ 8:1 to ≤ 15:1.

According to a preferred embodiment the Phosphine in the Ruthenium-Phosphine-Complex is a Tri(hetero)aryl and/or Bi(hetero)arylalkyl-Phosphine. These compounds have proven themselves in practice.

Generic group definition: Throughout the description and claims generic groups have been used, for example alkyl, alkoxy, aryl. Unless otherwise specified the following are preferred groups that may be applied to generic groups found within compounds disclosed herein:
alkyl: linear and branched Cl-C8-alkyl,
alkenyl: C2-C6-alkenyl,
cycloalkyl: C3-C8-cycloalkyl,
alkoxy: Cl-C6-alkoxy,
alkylene: selected from the group consisting of: methylene; 1,1-ethylene; 1,2-ethylene; 1,1-propylidene; 1,2-propylene; 1,3- propylene; 2,2-propylidene; butan-2-ol-1,4-diyl; propan-2-ol-1,3-diyl; 1, 4-butylene; cyclohexane-1,1-diyl; cyclohexan-1,2-diyl; cyclohexan-1,3- diyl; cyclohexan-1,4-diyl; cyclopentane-1,1-diyl; cyclopentan-1,2-diyl; and cyclopentan-1,3-diyl,
aryl: selected from homoaromatic compounds having a molecular weight under 300,
arylene: selected from the group consisting of: 1,2-phenylene; 1,3- phenylene; 1,4-phenylene; 1,2-naphtalenylene; 1,3-naphtalenylene; 1,4- naphtalenylene; 2,3-naphtalenylene; 1-hydroxy-2,3-phenylene; 1-hydroxy-2,4- phenylene; 1-hydroxy-2,5- phenylene; and 1-hydroxy-2,6-phenylene,
heteroaryl: selected from the group consisting of: pyridinyl; pyrimidinyl; pyrazinyl; triazolyl; pyridazinyl; 1,3,5-triazinyl; quinolinyl; isoquinolinyl; quinoxalinyl; imidazolyl; pyrazolyl; benzimidazolyl; thiazolyl; oxazolidinyl; pyrrolyl; carbazolyl; indolyl; and isoindolyl, wherein the heteroaryl may be connected to the compound via any atom in the ring of the selected heteroaryl,

Unless otherwise specified the following are more preferred group restrictions that may be applied to groups found within compounds disclosed herein:
alkyl: linear and branched C1-C6-alkyl,
alkenyl: C3-C6-alkenyl,
cycloalkyl: C6-C8-cycloalkyl,
alkoxy: C1-C4-alkoxy,
alkylene: selected from the group consisting of: methylene; 1,2-ethylene; 1,3-propylene; butan-2-ol-1,4-diyl; 1,4-butylene; cyclohexane-1,1-diyl; cyclohexan-1,2-diyl; cyclohexan-1,4-diyl; cyclopentane-1,1-diyl; and cyclopentan-1,2-diyl,
aryl: selected from group consisting of: phenyl; biphenyl; naphthalenyl; anthracenyl; and phenanthrenyl,
arylene: selected from the group consisting of: 1,2-phenylene; 1,3- phenylene; 1,4-phenylene; 1,2-naphtalenylene; 1,4-naphtalenylene; 2,3- naphtalenylene and 1-hydroxy-2,6-phenylene,
heteroaryl: selected from the group consisting of: pyridinyl; pyrimidinyl; quinolinyl; pyrazolyl; triazolyl; isoquinolinyl; imidazolyl; and oxazolidinyl, wherein the heteroaryl may be connected to the compound via any atom in the ring of the selected heteroaryl, heteroarylene: selected from the group consisting of: pyridin 2,3-diyl; pyridin-2,4-diyl; pyridin-2,6-diyl; pyridin-3,5-diyl; quinolin-2,3-diyl; quinolin-2,4-diyl; isoquinolin-1,3-diyl; isoquinolin-1,4-diyl; pyrazol-3,5-diyl; and imidazole-2,4-diyl.

According to a preferred embodiment of the present invention, the Ruthenium-Phosphine-Complex comprises more than one Phosphine, i.e. that in the coordination sphere of the ruthenium two or more trivalent phosphororganic components are present so that bonds (may it be covalent or coordination bonds) between the ruthenium and the phosphororganic components are formed at least temporarily during the reaction. Especially preferred are Ruthenium-Triphosphine-Complexes.

It should be noted that the present invention is not limited to Ruthenium-Phosphine-Complexes where all phosphines are bound to the Ruthenium. Actually in many applications of the present invention, the phosphine is used in excess so that also non-bound phosphines are present.

According to a preferred embodiment of the present invention, the Ruthenium-Phosphine-Complex comprises a bisphosphororganic component, trisphosphororganic component or a higher phosphororganic component. The term "bisphosphororganic component" and "trisphosphororganic component" in this context especially means and/or includes organic components in which two and three, respectively, trivalent phosphors are present. It should be noted that not necessarily all of the phosphines are bound to the Ruthenium during step a). Especially if higher phosphororganic components (in the sense of the present invention organic compounds with more than three trivalent phosphors) are used, not all of the phosphors are catalytically involved in the reaction; nevertheless these compounds are preferred compounds within the present invention as well.

Especially preferred in this context are phosphororganic components where the "bridging" moiety between the phosphors is an alkyl or alkylene moiety whereas the further ligands at the phosphor are aryl or heteroaryl. An especially preferred component in this context is Triphos = 1,1,1-tris(diphenylphosphinomethyl)ethane, which has the following structure:

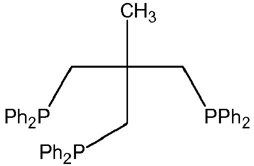

According to a preferred embodiment of the present invention, the Ruthenium-Phosphine-Complex comprises a bisphosphororganic component, trisphosphororganic component or a higher phosphororganic component which furthermore includes one or more donor moeites which can serve as ligands for the Ruthenium. Especially preferred in this context are carbenes, nitrogen containing-ligands such as amines or amides, phosphites, phosphoamidites, phosphoric ethers or esters. These compounds have proven themselves in practice. Yet especially preferred are bisphosphororganic components with one further donor moeity.

It should be noticed that according to one preferred embodiment of the present invention, the Ruthenium-Phosphine-complex may (prior to the reaction) comprise one or more "volatile" or easy removable ligand which stabilizes the complex so that it may be handled before the reaction but during the reaction sequence is replaced by the reactants. Suitable ligands are i.e. trimethylmethane, cyclopentadienyl, allyl, methylallyl, ethylene, cyclooctadiene, acetylactonate, acetate or carbon monoxide.

According to a preferred embodiment of the present invention, step a) is performed under acidic conditions whereby the acid is selected out of the group comprising organic or inorganic acids, especially sulfonic acids, especially methanesulfonic acid, trifluormethansulfonic acid, p-toluolsulfonic acid, p-bromobenzosulfonic acid, p-nitrobenzosulfonic acid, sulfuric acid, hydrochloric acid, hydrofluoric acid, trifluoracetic acid, perchloric acid or mixtures thereof. Even more preferred are acids which provide weak coordinating anions after deprotonation, such as bis(trifluoromethane)sulfonimide or mixtures thereof with aforementioned acids. These compounds have proven themselves in practice.

According to a preferred embodiment of the present invention, step a) is carried out at a temperature of ≥0°C to ≤200°C, preferably ≥20°C to ≤190°C, more preferred ≥60°C to ≤180°C, even more preferred ≥100°C to ≤170°C and most preferred at ≥120°C to ≤160°C. This has been shown to be most efficient for most applications within the present invention,

According to a preferred embodiment of the present invention, step a) is carried out in a dipolar protic or aprotic solvent or in CO₂. Preferred solvents are ethers (also cyclic ethers such as THF), alcohols, preferably ethanol or methanol and CO₂ (either liquid or near or supercritical). CO₂ is insofar a preferred solvent since it is the educt.

According to a preferred embodiment of the present invention, step a) is carried out at an initial hydrogen pressure of ≥1 bar, preferably ≥10 bar and most preferred ≥20 bar. This has been shown to greatly increase the reaction speed and efficiency for most applications of the present invention.

It is especially preferred that step a) is carried out at an initial CO₂ pressure of ≥1 bar, preferably ≥5 bar and most preferred ≥10 bar. This has been shown to greatly increase the reaction speed and efficiency for most applications of the present invention, too.

According to a preferred embodiment of the present invention, the method furthermore comprises a step a0) to be performed before step a):
a0) Reacting suitable precursor compounds to form the Ruthenium-Phosphine-Complex Suitable Ruthenium-containing precursor compounds include Ru(acac)₃, [Ru(cod)(methylallyl)₂] Ru(nbd)(methylallyl)₂, Ru(ethylene)2(methylallyl)₂.

Step a0) may be carried out at room temperature or at the same temperature at step a).

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the subclaims and the following description of the respective Examples which are for illustration of the invention only and non-binding.

### Examples

For complex synthesis and catalytic experiments, moisture and oxygen were excluded by working in a glove box or by using Schlenk techniques. Argon 4.8 (Messer, Germany) was used as inert gas. Glassware were dried under vacuum under heating with a heat gun, evacuated and refilled with argon at least three times. All solvents were purified by distillation prior to use. Toluene and pentane were degassed by bubbling argon with a frit, dried by passing over activated alumina in steel columns and stored over molecular sieves. All reagents were commercially available and used as received unless stated otherwise. Reaction gases hydrogen (5.0) and carbon dioxide (4.6) were supplied by Linde and PraxAir and used without further purification.

### Synthesis of [Ru(triphos)(tmm)]:

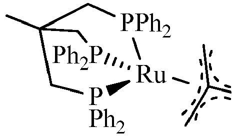

A 35 mL Schlenk tube was charged with 159.5 mg (0.5 mmol) [Ru(cod)(methylallyl)2] and 312.0 mg (0.5 mmol) 1,1,1-tris(diphenylphosphinomethyl)ethane (Triphos) in 25 mL toluene. After heating for 2 h at 110 °C, the resulting solution was concentrated in vacuo and treated with 10 mL of pentane. The precipitating complex was isolated and washed three times with 10 mL pentane. After drying, [Ru(triphos)(tmm)] was obtained as a bright yellow powder in 75 % yield.

### General procedures for the methylation of ammonia with CO₂/H₂.

All high pressure batch experiments were conducted in stainless steel 20 mL autoclaves equipped with a glass inlet and a magnetic stir bar. Prior to use, the autoclave was dried under vacuum for 3 hours and repeatedly filled with argon. Under an argon atmosphere, catalyst [Ru(triphos)(tmm)] (0.0125 g, 0.016 mmol), Al(OTf)₃ (0.061 g, 0.13 mmol), ammonia solution in dioxane (0.32 M, 2 mL), were mixed in a Schlenk tube. The mixture was transferred under argon via cannula to the autoclave. The autoclave was pressurized at room temperature with CO₂ to 20 bar and then H₂ was added up to a total pressure of 80 bar. The reaction mixture was stirred and heated to 150 °C using a preheated aluminum cone. After 24h, the autoclave was cooled to room temperature and kept stirring for 2 hours. The autoclave was then cooled in an ice bath and carefully vented. The reaction solution was analyzed by ¹H and ¹³C NMR with internal standard mesitylene.

The results of several examples are shown in Table 1 below:

**Table 1**

| Entry | Solvent | Temp [°C] | Time [h] | Yield |
|---|---|---|---|---|
| 1 | Dioxane | 150 | 12 | 21 |
| 2 | Dioxane | 150 | 15 | 30 |
| 3 | Dioxane | 120 | 20 | 16 |
| 4 | Dioxane | 180 | 20 | 34 |
| 5 | THF | 150 | 24 | 52 |
| 6 | Dioxane/H2O | 150 | 24 | 53 |

In reaction 5 NH₃ in THF 0.4 M (2 ml) was used, in reaction 6, 0.5 ml of H2O were added to the reaction mixture.

### General procedures for the methylation of ammonium chloride with CO₂/H₂.

All high pressure batch experiments were conducted in stainless steel 20 mL autoclaves equipped with a glass inlet and a magnetic stir bar. The pre-evacuated 20 mL autoclave was charged under argon with 1.0 mL of a 2 M NH₄Cl aqueous solution. Under an argon atmosphere, catalyst [Ru(triphos)(tmm)] (0.039 g, 0.05 mmol) was dissolved in 4 mL of degassed dioxane in a Schlenk tube. The mixture was then transferred under argon via cannula to the autoclave. The autoclave was subsequently pressurized at room temperature with CO₂ to 20 bar and then H₂ was added up to a total pressure of 80 bar. The reaction mixture was stirred and heated to 150 °C using a preheated aluminum cone. After 24h, the autoclave was cooled to room temperature and kept stirring for 2 hours. The autoclave was then cooled in an ice bath and carefully vented. The reaction solution was analyzed by ¹H and ¹³C NMR using mesitylene as internal standard.

The results of several examples are shown in the following Table 2:

**Table 2**

| Entry | [Ru] [mol%] | CO₂ [bar] | H₂ [bar] | Yield |
|---|---|---|---|---|
| 1 | 2.5 | 20 | 60 | 46 |
| 2 | 2.5 | 30 | 90 | 60 |
| 3 | 2.5 | 40 | 90 | 88 |
| 4 | 5 | 40 | 90 | > 99 |

In reaction 3, a total of 2 ml of H₂O was used.

One can see that the yields of trimethylamine are often excellent, sometimes nearly quantitative.

## Claims

1. A method for the synthesis of trimethylamine, comprising the step of:
a) hydrogenating CO₂ in the presence of ammonia and/or an ammonia salt and in the presence of a Ruthenium-Phosphine-complex, whereby when ammonia is used additionally a Lewis acid is present

2. The method according to claim 1, wherein the initial ratio of CO₂ to ammonia/ammonia salt (mol:mol) is ≥ 4:1, preferably ≥ 5:1

3. The method according Claim 1 or 2, wherein the the (initial) molar ratio of ruthenium to ammonia/ammonia salt is ≥0.01 to ≤0.1.

4. The method according to one of claims 1 to 3, wherein step a) is performed under acidic conditions

5. The method according to one of claims 1 to 4, wherein the Ruthenium-Phosphine-Complex comprises a bisphosphororganic component, trisphosphororganic or higher phosphororganic component

6. The method according to claim 5, wherein the Phosphine in the Ruthenium-Phosphine-Complex is a Tris(hetero)aryl and/or Bis(hetero)arylalkyl Phosphine.

7. The method according to one of claims 1 to 6, wherein step a) is performed under acidic conditions whereby the (initial) concentration of acid is ≥0.5 to ≤20 times the concentration of Ruthenium (in mol:mol).

8. The method according to one of claims 1 to 7, wherein step a) is performed under acidic conditions whereby the acid is selected out of the group comprising sulfonic acids, especially methanesulfonic acid, trifluormethansulfonic acid, p-toluolsulfonic acid, p-bromobenzosulfonic acid, p-nitrobenzosulfonic acid, sulfuric acid, hydrochloric acid, hydrofluoric acid, trifluoracetic acid, perchloric acid, bis(trifluoromethane)sulfonimide or mixtures thereof

9. The method according to any of the claims 1 to 8, wherein step a) is carried out at an initial hydrogen pressure of ≥1 bar.

10. The method according to any of the claims 1 to 9, wherein step a) is carried out in a dipolar protic or aprotic solvent or in CO₂.

## Patentansprüche

1. Verfahren zur Synthese von Trimethylamin, mit dem Schritt:
a) Hydrieren von CO₂ in Gegenwart von Ammoniak und/oder eines Ammoniaksalzes und in Gegenwart eines Ruthenium-Phosphin-Komplexes, wobei bei Verwendung von Ammoniak zusätzlich eine Lewis-Säure vorhanden ist.

2. Verfahren nach Anspruch 1, wobei das anfängliche Verhältnis von CO₂ zu Ammoniak/Ammoniaksalz (Mol:Mol) ≥ 4:1, vorzugsweise ≥ 5:1 ist.

3. Verfahren nach Anspruch 1 oder 2, wobei das (anfängliche) Molverhältnis von Ruthenium zu Ammoniak/Ammoniaksalz ≥ 0,01 bis ≤ 0,1 ist.

4. Verfahren nach einem der Ansprüche 1 bis 3, wobei Schritt a) unter sauren Bedingungen ausgeführt wird.

5. Verfahren nach einem der Ansprüche 1 bis 4, wobei der Ruthenium-Phosphin-Komplex eine bisphosphororganische Komponente, eine trisphosphororganische oder eine höhere phosphororganische Komponente aufweist.

6. Verfahren nach Anspruch 5, wobei das Phosphin in dem Ruthenium-Phosphin-Komplex ein Tris(hetero)aryl- und/oder Bis(hetero)arylalkylphosphin ist.

7. Verfahren nach einem der Ansprüche 1 bis 6, wobei Schritt a) unter sauren Bedingungen ausgeführt wird, wobei die (anfängliche) Säurekonzentration ≥ 0,5 Mal bis ≤ 20 Mal so groß ist wie die Rutheniumkonzentration (in Mol:Mol).

8. Verfahren nach einem der Ansprüche 1 bis 7, wobei Schritt a) unter sauren Bedingungen ausgeführt wird, wobei die Säure aus der Gruppe ausgewählt wird, die Sulfonsäuren, insbesondere Methansulfonsäure, Trifluormethansulfonsäure, p-Toluolsulfonsäure, p-Brombenzosulfonsäure, p-Nitrobenzosulfonsäure, Schwefelsäure, Salzsäure, Flusssäure, Trifluoressigsäure, Perchlorsäure, Bis(trifluormethan)sulfonimid oder Gemische davon aufweist.

9. Verfahren nach einem der Ansprüche 1 bis 8, wobei Schritt a) bei einem anfänglichen Wasserstoffdruck von ≥ 1 bar ausgeführt wird.

10. Verfahren nach einem der Ansprüche 1 bis 9, wobei Schritt a) in einem dipolaren protischen oder aprotischen Lösungsmittel oder in CO₂ ausgeführt wird.

## Revendications

1. Procédé de synthèse de triméthylamine, comprenant les étapes suivantes :
a) l'hydrogénation de CO₂ en présence d'ammoniac et/ou d'un sel d'ammonium et en présence d'un complexe-Ruthénium-Phosphine, dans lequel lorsque l'ammoniac est utilisé, un acide de Lewis est additionnellement présent.

2. Procédé selon la revendication 1, dans lequel le ratio initial de CO₂ à ammoniac/sel d'ammonium (mol : mol) est ≥ 4 : 1 , préférablement ≥ 5 : 1.

3. Procédé selon la revendication 1 ou la revendication 2, dans lequel le ratio molaire (initial) de ruthénium à ammoniac/sel d'ammonium est ≥ 0,01 et ≤ 0,1.

4. Procédé selon l'une des revendications 1 à 3, dans lequel l'étape a) est effectuée dans des conditions acides.

5. Procédé selon l'une des revendications 1 à 4, dans lequel le complexe-Ruthénium-Phosphine comprend un composé bis-phosphore organique, un composé tris-phosphore organique ou phosphore organique d'ordre supérieur.

6. Procédé selon la revendication 5, dans lequel le complexe-Ruthénium-Phosphine est un Tris(hétéro)aryle et/ou Bis(hétéro)arylalkyle Phosphine.

7. Procédé selon l'une des revendications 1 à 6, dans lequel l'étape a) est effectuée dans des conditions acides où la concentration (initiale) d'acide est ≥ 0,5 et ≤ 20 fois la concentration de Ruthénium (en mol : mol).

8. Procédé selon l'une des revendications 1 à 7, dans lequel l'étape a) est effectuée dans des conditions acides où l'acide est sélectionné parmi le groupe comprenant les acides sulfoniques, particulièrement l'acide méthanesulfonique, l'acide trifluorométhanesulfonique, l'acide p-bromobenzènesulfonique, l'acide p-nitrobenzènesulfonique, l'acide sulfurique, l'acide chlorhydrique, l'acide trifluoroacétique, l'acide perchlorique, la bis(trifluorométhane) sulfonimide ou des mélanges de ceux-ci.

9. Procédé selon l'une des revendications 1 à 8, dans lequel l'étape a) est effectuée à une pression d'hydrogène initale ≥ 1 bar.

10. Procédé selon l'une des revendications 1 à 9, dans lequel l'étape a) est effectuée dans un solvant dipolaire protique ou aprotique, ou dans le CO₂.
